# EUROPÄISCHE PATENTSCHRIFT

(11) **EP 1 785 163 B1**
(45) Veröffentlichungstag und Bekanntmachung des Hinweises auf die Patenterteilung: **28.05.2008**
(21) Anmeldenummer: 06077024.5
(22) Anmeldetag: 13.11.2006
(51) Int. Cl.: A61N 7/00

(54) **Ultraschallbehandlungseinheit**
Ultrasound treatment unit
Unité de traitement par ultrasons

(30) Priorität: 14.11.2005 DE 202005017857 U
(43) Veröffentlichungstag der Anmeldung: 16.05.2007
(73) Patentinhaber: BANDELIN electronic GmbH & Co. KG, 12207 Berlin (DE)
(72) Erfinder: Kähler, Sebastian, 13187 Berlin (DE); Jung, Rainer, 13125 Berlin (DE); Helke, Juliane, 12203 Berlin (DE); Radandt, Roland, 10407 Berlin (DE)
(74) Vertreter: Pfenning, Meinig & Partner GbR

(56) Entgegenhaltungen:
- EP-A- 0 346 496
- DE-A1- 10 153 126
- US-A1- 2003 135 084

## Beschreibung

Gegenstand der Erfindung ist eine Behandlungseinheit zum Einbringen von Niederfrequenz-Ultraschall in biologisches Gewebe, vorzugsweise in ausgewählten Bereichen des menschlichen Körpers, für die medizinische Behandlung von muskuloskelettalen Krankheitsbildern, Weichteilverletzungen, Verletzungen von Sehnen und Bändern, Neuralgien u.a., aber auch zur Wundheilungsstimulation und dermatokosmetischen Zwecken gemäß der vorliegenden konstruktiven Ausführung und aufgeführten Schutzansprüche. Die Behandlungseinheit ist außerdem für in-vitro-Anwendungen geeignet.

Vorrichtungen für die medizinische Anwendung von Ultraschall, wie auch Niederfrequenz-Ultraschall, bestehen in der Regel aus einem separaten Generatorteil zur Erzeugung von elektrischer Hochfrequenzenergie und Mitteln zur Bedienung und Parametereinstellung sowie einer ebenfalls separaten Behandlungseinheit mit einem Ultraschallerzeuger zur Übertragung der Ultraschallschwingungen auf und in das biologische Gewebe. Für die Umwandlung der elektrischen hochfrequenten Schwingungen in Schallschwingungen im Ultraschallbereich werden vorzugsweise piezoelektrische Wandlerelemente eingesetzt. Die Generatorfrequenz ist an die bevorzugte Eigenfrequenz des elektroakustischen Ultraschallerzeugers angepasst.

In der Regel ist eine Behandlungseinheit für das Einbringen von Ultraschallenergie in biologisches Gewebe als Handvorrichtung ausgeführt und besteht aus einem Ultraschallerzeuger, der in einem ein- oder mehrteiligen, offenen oder in sich geschlossenen Gehäuse untergebracht ist. Je nach Ausführungsart hat der Ultraschallerzeuger direkten Kontakt zum biologischen Gewebe oder ist von diesem durch eine Zwischenschicht, die metallisch oder nichtmetallisch oder auch fest oder flüssig ausgeführt sein kann, getrennt.

Die bekannten Konstruktions- und Ausführungsformen der Behandlungseinheiten, die mit Frequenzen im Bereich von 1 MHz oder höher arbeiten, können für den Niederfrequenz-Ultraschall auf Grund der hier nötigen, größeren Abmessungen der Piezokeramiken nur bedingt übernommen werden. Es werden deshalb Ultraschallerzeuger bevorzugt, die als sogenannte DML-Systeme (Double Mass Loaded) oder Sandwich-Systeme ausgeführt sind.

In der Offenlegungsschrift DE 101 53 126 A1 ist eine typische Ausführungsform einer Behandlungseinheit für eine manuelle Anwendung mit einem DML-System als Ultraschallerzeuger beschrieben. Kennzeichnend ist eine Befestigung und Verbindung des Ultraschallerzeugers mit dem Gehäuse im schwingungsbehafteten Bereich seines schallaktiven Teiles, die durch Einpressen und Fixierung des Ultraschallerzeugers mittels O-Ringen realisiert wird. Dies hat eine mehr oder weniger starke Übertragung von Ultraschallenergie auf das topfförmige Gehäuse zur Folge. Das führt zum einen zu einer Bedämpfung des Ultraschallerzeugers, die durch eine erhöhte Zufuhr an elektrischer Energie ausgeglichen werden muss und zum anderen zur Notwendigkeit zusätzlicher Schutzmaßnahmen, um zu verhindern, dass die auf das Gehäuse übertragene Ultraschallenergie auf Körperteile des Anwenders, z. B. die Hand, übergehen und in diese eindringen kann. Zur Minderung wird in den Ausführungsbeispielen eine akustische Entkopplung des topfförmigen Gehäuses vom Ultraschallerzeuger durch einen Ring aus elastischem Material oder einem gleichartigen Überzug des Gehäuses vorgeschlagen.

Darüber hinaus führt diese Gehäusebefestigung des Ultraschallerzeugers zu einer inhomogenen Schallabstrahlung, d. h. durch die Bedämpfung bzw. Abbremsung der Schwingungen im Außenbereich des Ultraschallerzeugers ergibt sich ein Schallfeld, welches von einem mittigen Maximum zu den Randbereichen deutlich abnimmt. Stärke und Verlauf der Intensitätsverteilung sind dabei stark abhängig von der jeweils gewählten konstruktiven Ausführung. Dies ist für eine therapeutische Anwendung ungünstig, da es zu lokalen Überhitzungen kommen kann und eine Intensitätsreduzierung notwendig macht, die ggf. das therapeutische Ergebnis ungünstig beeinflusst.

Aufgabe der Erfindung ist die Konstruktion und Herstellung einer robusten und leicht handhabbaren Behandlungseinheit zur Durchführung medizinischer Behandlungen mittels Niederfrequenz-Ultraschall, vorzugsweise im Bereich der Physikalischen Therapie, wobei die Behandlungseinheit manuell geführt und direkten Kontakt zum biologischen Gewebe hat.

Diese Aufgabe wird durch eine Behandlungseinheit nach Anspruch 1 gelöst.

Erfindungsgemäß gegeben ist eine Behandlungseinheit für therapeutische sowie in-vitro-Anwendungen, enthaltend: ein topfförmiges Gehäuse sowie einen im Wesentlichen stabförmigen Ultraschallerzeuger, wobei das topfförmige Gehäuse eine Aufnahme mit zwei Dichtungen aufweist, und der Ultraschallerzeuger einen Flansch aufweist, welcher zumindest eine zu einer Querschnittsebene des Ultraschallerzeugers unter einen Winkel 5° < α < 85° winklige oder gekrümmte Schrägfläche aufweist, wobei mindestens eine Dichtung auf der Schrägfläche des Flansches abgestützt ist.

Hierdurch ergeben sich erfindungsgemäß mehrere Vorteile. Zum einen nimmt der Flansch die gesamte Kraft, die auf den Ultraschallerzeuger von dem zu behandelnden Objekt aus wirkt, auf.

Unter "topfförmiges Gehäuse" wird jegliche Art von Gehäuse verstanden, welche einen vorzugsweise stabförmigen Ultraschallerzeuger aufnehmen kann. Es muss sich hierbei beispielsweise nicht um einen klassischen "Topf" handeln, dieser Topf kann auch zahlreiche Öffnungen bzw. Aussparungen bzw. fehlende Wände aufweisen, wichtig ist hier lediglich die Funktion der Einfassung des Ultraschallerzeugers. Mit "im Wesentlichen stabförmig" ist bei dem Ultraschallerzeuger, welcher primär längsschwingend ist zur Behandlung einer bestimmten Partie, z.B. Hautpartie, dass dieser eine gewisse Ausdehnung in Längsrichtung hat, gemeint. Hierdurch ist jedoch nicht zwingend gemeint, dass hier die Länge größer ist als beispielsweise der Durchmesser dieses Ultraschallerzeugers.

Hierbei sind Ultraschallerzeuger und Gehäuse nicht "starr" aneinander gekoppelt, sonst käme es zu einer Energieentziehung aus der Behandlungseinheit heraus, welche die Effektivität der Behandlungseinheit schmälern würde. Außerdem wird hierdurch eine "Verstimmung" des Systems vermieden.

Besonders wichtig ist die Zuordnung der Schrägfläche zu der daran angrenzenden Dichtung. Der Flansch variiert in seiner Größe mit jeder Schwingung, selbst wenn er in der Nulllage der Längsschwingung angeordnet ist, in Durchmesser und Dicke. Dadurch entsteht eine (sehr geringe, sich im µm-Bereich bewegende) Relativbewegung. Durch die erfindungsgemäße Anordnung der Schrägfläche mit einer hierzu kompatiblen Dichtung, welche beispielsweise "abrollt" bzw. "abgleitet" wird der Flansch optimal geführt, ohne hierbei zu verklemmen.

Hierdurch sind Abdichtung und Bewegungsausgleich unter Belastung in hohem Maße gegeben.

Vorteilhafte Weiterbildungen der vorliegenden Erfindung werden in abhängigen Ansprüchen angegeben.

Eine vorteilhafte Weiterbildung sieht vor, dass der Flansch seitlich umlaufend ist. Hierdurch wird es möglich, den Ultraschallerzeuger beispielsweise in einem einfachen spanabhebenden Verfahren, z.B. Drehen, herzustellen.

Besonders vorteilhaft ist, dass der Flansch in einer bzw. um eine Nulllage der Längsschwingung des Ultraschallerzeugers angeordnet ist. Dies ist beispielsweise der Fall, wenn ein λ/4-Abstand bezüglich des nächstliegenden Endes des Ultraschallerzeugers gegeben ist. Hierdurch wird außerdem erreicht, dass Eigenbewegungen des Flansches möglichst gering sind, so dass auszugleichende Relativbewegungen im Bereich der Schrägflächen ebenfalls möglichst klein sind.

Eine weitere vorteilhafte Weiterbildung sieht vor, dass die Flanschdicke der Ungleichung λ/60 ≤ t ≤ λ/12 entspricht und/oder t ≥ 1 mm ist. Hierdurch wird erreicht, dass die Flanschdicke einerseits nicht so groß ist, dass eine zwangsweise Relativbewegung durch die große Ausdehnung in Längsrichtung gegeben ist. Auf der anderen Seite wird eine bestimmte Mindestdicke erreicht, welche eine notwendige mechanische Stabilität des Flansches gewährleistet. Es sei bemerkt, dass der Flansch nicht zwangsweise eine gleichbleibende Dicke haben muss und auch nicht mit seiner oberen und unteren Grenzfläche streng senkrecht zur Längsachse des Ultraschallerzeugers laufen muss. So sind beispielsweise im Querschnitt wellenförmige Anordnungen möglich. Vorzugsweise ist der gemittelte Durchmesser des Flansches 15 % bis maximal 150 % größer als der gemittelte Durchmesser des übrigen Ultraschallerzeugers.

Vorteilhafterweise weist die Schrägfläche, egal ob sie geradlinig flächig, gestuft oder gekrümmt ist, eine Längenausdehnung senkrecht zur Längsachse des Ultraschallerzeugers von 0,5 mm - 10 mm, vorzugsweise 0,5 mm - 4 mm auf. Hierdurch wird auch bei stärkeren Relativbewegungen ein fester Halt selbst bei starker Belastung des Flansches und starken Schwingungen gewährleistet.

Eine weitere vorteilhafte Weiterbildung sieht vor, dass bei einer geradlinigen Schrägfläche der Winkel α zwischen Schrägfläche und Fläche senkrecht zur Ultraschallerzeugerlängsachse α = 10° - 80°, vorzugsweise α = 30° - 60°, besonders vorzugsweise α = 30° - 45° beträgt.

Unabhängig hiervon kann die Schrägfläche auch Stufen aufweisen bzw. eine Aneinanderreihung unterschiedlicher Winkel aufweisen bzw. gekrümmt sein bzw. sich ändernde Krümmungen aufweisen.

Eine weitere vorzugsweise Ausführungsform sieht vor, dass der Flansch zur Minimierung der Eigenresonanz perforiert ist.

Auch kann eine Verdrehsicherung zwischen dem Ultraschallerzeuger und dem Gehäuse gegeben sein. Dies wird vorzugsweise durch eine Nut-Stift-Anordnung gewährleistet.

Vorzugsweise ist die Aufnahme im Gehäuse nutförmig umlaufend ausgestaltet. Hierzu korrespondierend sind vorzugsweise auch die Dichtungen entsprechend umlaufend ausgeführt, dasselbe gilt für den Flansch. Hierdurch ist eine gute mechanische Festigkeit und außerdem eine Abdichtwirkung zum Schutz des Inneren des topfförmigen Gehäuses gegen Feuchtigkeitseintritt gegeben. Die Dichtungen haben hierbei vorzugsweise einen runden, ellipsoiden oder mehreckigen Querschnitt, da hierdurch eine Relativbewegung zu den Schrägflächen besonders gut möglich ist.

Es ist außerdem vorteilhaft, dass die Gesamtlänge des Ultraschallerzeugers ein ganzzahliges Vielfaches von λ/2 in seiner Längsschwingungsrichtung ist. Vorzugsweise ist der Ultraschallerzeuger hierbei mehrteilig ausgeführt, wobei vorzugsweise ein oder mehrere scheibenförmige Piezoelemente zwischen einem Oberteil und einem zur Berührung mit dem zu behandelnden Objekt in Kontakt zu bringendem Unterteil vorgesehen sind. Vorteilhafte Weiterbildungen des Unterteiles sehen vor, dass dieses neben der beschriebenen Stabform auch kegelförmig, tellerförmig oder anderweitig ausgeführt sein kann. Das Unterteil sollte hierbei zumindest bereichsweise aus einem biokompatiblen Material, vorzugsweise Titan oder einer Titanlegierung bestehen. Das Gehäuse kann vorzugsweise aus einem Kunststoff, besonders vorzugsweise aus Polyoxymethylen (POM) hergestellt sein.

Eine weitere Ausführungsform betrifft die Gestaltung des Unterteils des Ultraschallerzeugers.

Bei einem stabförmigen Unterteil des Ultraschallerzeugers kann es an der Abstrahlungsfläche des Handapplikators zu einer unerwünschten Erwärmung der Hautoberfläche kommen. Dieser Effekt wird zusätzlich verstärkt durch die Tatsache, dass der Ultraschallerzeuger in der Praxis kein idealer Kolbenschwinger mehr ist und dadurch keine homogene Schallabstrahlung im biologischen Gewebe gewährleistet, sondern die Intensität vom Zentrum zu den Außenbereichen abfällt. Eine weitere Aufgabe der Erfindung ist es daher, ausgehend von den bekannten Ausführungsformen den Ultraschallerzeuger im Handapplikator so zu gestalten, dass eine Verlängerung des Nahfelds und eine weitgehend homogene Schallabstrahlung im biologischen Gewebe erreicht wird.

Die Aufgabe wird dadurch gelöst, dass gemäß einer weiteren vorteilhaften Weiterbildung die Schallaustrittsfläche stegförmig, vorzugsweise ringförmig ausgeformt ist und nur dieser stegförmige Bereich mit dem biologischen Gewebe in Kontakt kommt. Durch eine entsprechende konstruktive Ausgestaltung wird gewährleistet, dass auch bei Verwendung eines Koppelmediums, wie z. B. Ultraschallgel, nur vorzugsweise die aus diesem ringförmigen Bereich emittierte Ultraschallenergie in das biologische Gewebe übertragen wird. Zur Gewährleistung der Biokompatibilität wird der mit dem biologischen Gewebe in Kontakt tretende Bereich des Schwingungserzeugers aus einer geeigneten Titanlegierung hergestellt.

Der Handapplikator besteht demnach z.B. aus einem topfförmigen Gehäuse mit einem verlustarm eingefassten Ultraschallerzeuger, der in bekannter Weise als ein DML-System ausgeführt ist; bestehend aus einer Gegenmasse, Piezokeramiken und einem schallaktiven Unterteil, die miteinander fest verschraubt sind. Gegenmasse und schallaktives Unterteil sind vorzugsweise rotationssymmetrische Teile und bestehen aus üblichen dämpfungsarmen Werkstoffen. Das sich an die Piezokeramiken anschließende schallaktive Unterteil weist im dehnungsfreien Bereich λ/4 für die Längsauslenkung einen dünnwandigen Flansch mit größerem Durchmesser auf als die Gegenmasse, die Piezokeramiken und das schallaktive Unterteil besitzen, wodurch auf den Flansch nur eine geringfügige Querauslenkung wirkt. Der Flansch dient der Befestigung des Ultraschallerzeugers im topfförmigen Gehäuse. Beim aktiven Betreiben des Ultraschallerzeugers wird durch geeignete konstruktive Maßnahmen, z. B. ein Gleiten des Flansches auf O-Ringen, eine Energieübertragung auf das Gehäuse verhindert oder zumindest deutlich minimiert, so dass keine weiteren speziellen Dämpfungsmaßnahmen zur akustischen Entkopplung des Handapplikators zum Schutz des Anwenders erforderlich sind. Der Kontaktbereich des schallaktiven Unterteils zum biologischen Gewebe enthält in seinem Zentrum eine muldenförmige Ausarbeitung mit definierter Tiefe, so dass eine ringförmige Struktur gebildet wird und nur die Fläche der ausgebildeten Ringstruktur den Kontakt zum biologischen Gewebe herstellt.

Durch die ringförmige Abstrahlungsfläche des Handapplikators werden im aktiven Betrieb Wellenfronten gebildet, so dass sich nicht wie bei der bekannten Ausführung das Fernfeld unmittelbar, sondern erst nach Überlagerung der Wellenfronten ausbildet. Auf diese Weise wird das sich ausbildende Intensitätsmaximum von der Kontaktfläche tiefer in das biologische Gewebe hin verschoben, wobei der resultierende Abstand abhängig von der Wellenlänge im Gewebe und der konstruktiven Ausführung ist. Ebenso wird durch geeignete konstruktive Parameter der Ringstruktur erreicht, dass die Ringstruktur in ihrem Flächenbereich eine weitgehend homogene Abstrahlcharakteristik aufweist. Mit den genannten Maßnahmen wird in tiefergelegenen Bereichen des biologischen Gewebes eine Erhöhung der Ultraschallintensität erreicht und die Kontaktfläche des biologischen Gewebes thermisch weniger belastet. Die beschriebene Ausführungsform ist besonders gut geeignet für die Therapie muskulärer Schmerzsyndrome, insbesondere der myofaszialen Triggerpunkte.

Eine besonders vorteilhafte Weiterbildung sieht vor, dass das Unterteil des Ultraschallerzeugers in seinem Kontaktbereich zum zu behandelnden Objekt eine zumindest bereichsweise von einer stegförmigen Struktur umgebene muldenartige Ausarbeitung aufweist, welche sich in Längsachse des Ultraschallerzeugers vorzugsweise im Bereich von 1/4 bis 1/6 der Länge des Unterteils erstreckt. Dies ist also eine Abkehr von flachen (ebenen) Abstrahlungsflächen bzw. allmählich konvexen/konkaven Strukturen. Die stegförmige Struktur dient hierbei der Übertragung des Ultraschalls, wobei gegenüber einer ebenen Vollfläche die Abstrahlfläche bzw. die Richtcharakteristik verändert ist.

Die Projektionsfläche der muldenartigen Ausarbeitung beträgt senkrecht zur Längsachse des Ultraschallerzeugers vorzugsweise das 0,1-fache bis 0,5-fache des Kontaktbereichs des Ultraschallerzeugers.

Eine weitere vorteilhafte Weiterbildung des Unterteils vom Ultraschallerzeuger in seinem Kontaktbereich sieht vor, dass vorzugsweise sich umschließende, insbesondere konzentrische, und durch dazwischen liegende muldenartige Ausarbeitungen getrennte stegförmige Strukturen gegeben sind. Auch dies ist eine Abkehr von einer rein flächig/ebenen Abstrahlfläche, wobei durch die ineinander geschachtelten stegförmigen Strukturen jeweils vorteilhafte Richtcharakteristika für eine verbesserte Tiefenwirkung erzeugt werden können.

Unabhängig davon, ob lediglich eine stegförmige Struktur oder mehrere ineinander "geschachtelte" Strukturen gegeben sind, müssen diese nicht (wie beispielsweise in den Ausführungsbeispielen) kreisrund sein, sondern können auch ellipsoide, rechteckige oder dreieckige Formen in der Ebene senkrecht zur Längsachse des Ultraschallerzeugers (also bei einer Draufsicht auf das Unterteil vom "Patienten" aus) annehmen.

Weitere vorteilhafte Weiterbildungen befassen sich mit Art und Frequenz der durch den separaten Generator erzeugten hochfrequenten Wechselenergie für den Ultraschallerzeuger.

Eine Weiterbildung sieht vor, dass der Ultraschallerzeuger mit gepulster Hochfrequenzenergie anregbar ist und die Pulsationsfrequenz der Hochfrequenzenergie im Bereich von 1 - 100 Hz, bevorzugt zwischen 8 - 40 Hz, liegt.

Hierdurch werden die thermische Belastung des Gewebes reduziert und die mechanoakustischen Wirkeffekte verstärkt.

Bevorzugt ist die Einschaltzeit kleiner als die Ausschaltzeit der gepulsten Hochfrequenzenergie, besonders bevorzugt beträgt sie 10% - 50% der Ausschaltzeit.

Die Frequenz der dem Ultraschallerzeuger zugeführten Hochfrequenzenergie liegt vorzugsweise im Bereich zwischen 30 kHz - 120 kHz, bevorzugt zwischen 40 kHz und 60 kHz.

Weitere vorteilhafte Weiterbildungen werden in den übrigen Ansprüchen angegeben.

Im Folgenden wird der erfindungsgemäße Gedanke nochmals mit anderen Worten beschrieben.

Die Behandlungseinheit besteht aus einem topfförmigen Gehäuse mit einem verlustarm eingefassten Ultraschallerzeuger, der in bekannter Weise als ein DML-System ausgeführt ist; bestehend aus einer Gegenmasse, Piezokeramiken und einem schallaktiven Unterteil, die miteinander fest verschraubt sind. Gegenmasse und schallaktives Unterteil sind rotationssymmetrische Teile und bestehen aus üblichen dämpfungsarmen Werkstoffen, z.B. Titanlegierungen. Das sich an die Piezokeramiken anschließende schallaktive Unterteil weist im dehnungsfreien Bereich λ/4 für die Längsauslenkung einen dünnwandigen Flansch mit größerem Durchmesser auf als die Gegenmasse, die Piezokeramiken und das sich unmittelbar anschließende schallaktive Unterteil besitzen, wobei auf das Gehäuse der Behandlungseinheit über den Flansch nur eine sehr geringe Querauslenkung wirkt, die sich aus seiner vorgenannten Anordnung in Längsrichtung sowie seinem Durchmesser/Dickenverhältnis und seiner beidseitig abgeflachten Kontur am Außenrand ergibt.

Das topfförmige Gehäuse des Ultraschallerzeugers enthält eine ringförmige Nut, in welche der Flansch mit seiner abgeflachten Außenkontur zwischen zwei dort ebenfalls eingebrachten O-Ringen eingedrückt wird. Die beidseitig abgeflachte Kontur am Außenrand des Flansches folgt einer vorgegebenen schiefen Ebene, deren Winkel auf den Einpressdruck und die Kompressibilität der O-Ringe abgestimmt ist. Auf diese Weise wird ein direkter Kontakt des Ultraschallerzeugers zum Gehäuse sicher ausgeschlossen. Neben einer einfachen Abschrägung der Flanschdicke zum Außenrand hin kann die abgeflachte Kontur mittels einer abgestuften Abschrägung, mittels aufeinander folgenden Abschrägungen unterschiedlichen Winkels oder auch jeweils mit einer Übergangskontur nach einer mathematischen Funktion zwischen ihnen oder am Ende und Beginn, z. B. eines Radius, ausgeführt sein. Beim aktiven Betreiben des Ultraschallerzeugers ist dadurch eine geringe Korrekturbewegung oder auch Gleiten des Flansches auf den O-Ringen möglich und es wird eine Energieübertragung auf das Gehäuse verhindert oder zumindest so stark minimiert, dass keine weiteren speziellen Dämpfungsmaßnahmen zur akustischen Entkopplung der Behandlungseinheit zum Schutz des Anwenders erforderlich sind.

Im Betrieb und bei der Handhabung am Patienten durch den Anwender wird das Schwingungsverhalten des Ultraschallerzeugers nicht mehr beeinflusst und wird nur von der geometrischen Ausführung und den daraus resultierenden physikalischen Eigenschaften bestimmt. Weiterhin kann die Länge des schallaktiven Teils des Ultraschallerzeugers an die jeweilige Aufgabenstellung leicht angepasst werden unter Beachtung, dass die Gesamtlänge des Ultraschallerzeugers ein ganzzahliges Vielfaches von λ/2 in Längsschwingungsrichtung sein muss. So ist es z.B. mit einem verlängerten schallaktiven Teil möglich, Ultraschall in tiefere Körperhöhlen einzuleiten, wobei ggf. eine zusätzliche akustische Abschirmung vorzusehen ist.

Ein weiterer Vorteil der erfindungsgemäßen Ausführung ist die einfache Montage und Demontage des Ultraschallerzeugers zum topfförmigen Gehäuse, wodurch sich Herstellung und Service der Behandlungseinheit sehr vereinfachen und Kosten gesenkt werden.

Ein Ausführungsbeispiel der Erfindung ist in den Figuren dargestellt und wird in der nachfolgenden Beschreibung näher erläutert.
- Fig. 1: zeigt eine Behandlungseinheit 13 für therapeutische sowie in-vitro-Anwendungen. Diese weist ein Gehäuse 1 auf, welches topfförmig ausgeführt ist.
- Fig. 2: zeigt eine weitere Ausführungsform einer erfindungsgemäßen Behandlungseinheit.
- Fig. 3: zeigt einen Schnitt sowie eine perspektivische Ansicht eines Unterteils eines Ultraschallerzeugers.

Innerhalb dieses Gehäuses ist ein stabförmiger Ultraschallerzeuger 12 gehalten. Das topfförmige Gehäuse 1 weist hierbei eine Aufnahme 9 mit zwei Dichtungen 7, 8 auf, welche einen Flansch 6 des Ultraschallerzeugers von oben und von unten umlaufend unter Druck setzen und somit fixieren.

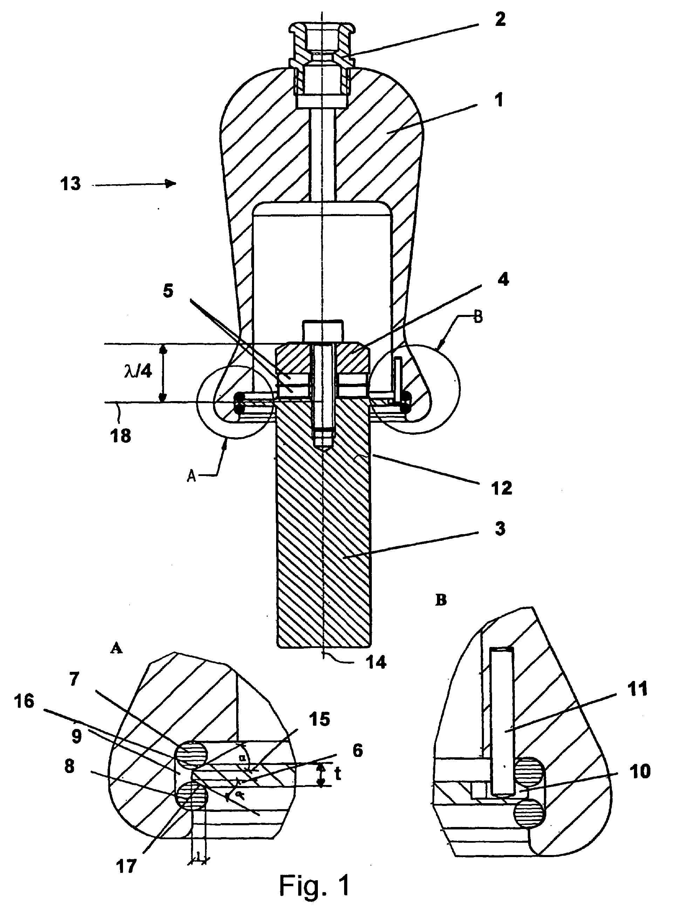

Bei dem in Figur 1 gezeigten montierten und betriebsfertigen Zustand der Behandlungseinheit ist ein Hohlraum im Inneren des topfförmigen Gehäuses 1 gegeben, welcher durch den Ultraschallerzeuger 12 bzw. dessen Flansch verschlossen wird. In diesem Bereich ist der antreibende, aktive Teil des Ultraschallerzeugers 12 in Form von zwei verschraubten, piezokeramischen Lochscheiben 5 angeordnet. Die elektrische Zuleitung zu diesen Piezokeramiken erfolgt durch die an der Oberseite des Gehäuses 1 gezeigte Kabelverschraubung 2. Zur Verhinderung eines Verdrehens der elektrischen Zuleitung zum Ultraschallerzeuger 12 ist eine Verdrehsicherung vorgesehen, siehe Detail B. Hierbei erstreckt sich ein im Gehäuse gehaltener Stift 11 in eine Ausfräsung 10 im Flansch 6 vom Unterteil 3. Durch diesen Formschluss kann eine Verdrehung um die Ultraschallerzeugerlängsachse 14 begrenzt bzw. ausgeschlossen werden.

Bevor auf weitere Einzelheiten der erfindungsgemäßen Behandlungseinheit eingegangen wird, wird zunächst kurz deren Montage beschrieben. Hierzu erfolgt nach Montage des Ultraschallerzeugers sowie Verdrahtung und Ziehen eines Kabels durch die Durchführung 2 hindurch sowie dem Einstecken eines Stiftes 11 in das Gehäuse 1 das Einlegen einer ersten Dichtung 7. Daraufhin wird der Ultraschallerzeuger mit seiner Flanschoberseite gegen diese Dichtung gepresst. Anschließend erfolgt ein Einschnappen der Dichtung 8, um somit den Flansch zu fixieren. Besonders vorteilhaft hieran ist, dass das Gehäuse einteilig ausgeführt werden kann.

Gezeigt ist also eine Behandlungseinheit 13 für therapeutische sowie in-vitro-Anwendungen, enthaltend:

Ein topfförmiges Gehäuse 1 sowie einen im Wesentlichen stabförmigen Ultraschallerzeuger 12, wobei das topfförmige Gehäuse 1 eine Aufnahme 9 mit zwei Dichtungen 7, 8 aufweist, und der Ultraschallerzeuger 12 einen Flansch 6 aufweist, welcher zumindest eine zu der Querschnittsebene 15 des Ultraschallerzeugers 12 unter einem Winkel 5° < α < 85° winklige oder gekrümmte Schrägfläche 16, 17 aufweist, wobei mindestens eine Dichtung 7, 8 auf der Schrägfläche 16, 17 des Flansches 6 abgestützt ist. Der Flansch ist hierbei seitlich umlaufend ausgeführt. Die korrespondierende Aufnahme 9 ist ebenfalls umlaufend ausgestaltet, dasselbe gilt für die Dichtungen 7, 8, welche vorliegend als im unbelasteten Zustand im Wesentlichen im Querschnitt als runde "O-Ringe" ausgeführt sind.

Der Ultraschallerzeuger hat eine solche Länge, dass diese einen ganzzahliges Vielfaches von λ/2 der Längsschwingungsrichtung darstellt. Der Ultraschallerzeuger 12 ist hierbei mehrteilig ausgeführt, wobei vorliegend scheibenförmige Piezoelemente 5 zwischen einem Oberteil 4 und einem zur Berührung mit dem zu behandelnden Objekt in Kontakt zu bringendes Unterteil 3 vorgesehen sind. Dieses Unterteil ist vorliegend aus einer Titanlegierung hergestellt. Das topfförmige Gehäuse 1 ist aus Gewichtsgründen bzw. Gründen der einfachen Herstellbarkeit bzw. Hygienegründen aus Kunststoff, vorliegend POM hergestellt.

Der erfindungsgemäße Flansch ist vorliegend um eine Nulllage der Längsschwingung des Ultraschallerzeugers angeordnet. Der Abstand vom oberen Ende des Ultraschallerzeugers beträgt λ/4. Auf halber Höhe des Flansches ist eine zur Blattebene sowie zur Ultraschallerzeugerlängsachse 14 senkrechte Fläche 18 gezeigt, welche die entsprechende Nulllage der Längsschwingung des Ultraschallerzeugers zeigt. Die Flanschdicke t (siehe Detail A) ist vorliegend t = 1,6 mm bzw. t = λ/44.

Der gemittelte Durchmesser des Flansches ist vorliegend 80 % größer als der gemittelte Durchmesser des übrigen Ultraschallerzeugers.

Besonders wichtig sind die erfindungsgemäßen Schrägflächen des Flansches sowie deren Anbindung an die Dichtungen.

Diese werden im Folgenden anhand vom Detail A näher erläutert.

Dort ist die Aufnahme 9 in Form einer ringförmigen Nut im topfförmigen Gehäuse gezeigt. Diese weist an ihrem oberen und ihrem unteren Ende Krümmungen auf, in welche die als O-Ringe ausgeführten Dichtungen 7 bzw. 8 formschlüssig eingreifen. Zwischen den Dichtungen ist der Flansch 6 eingeklemmt. Die Schrägflächen 16 bzw. 17 des Flansches sind vorliegend als gerade Schrägflächen unter einem konstanten Winkel α ausgeführt. Der Winkel α beträgt für beide Schrägflächen gegenüber der Querschnittsfläche, welche senkrecht auf der Ultraschallerzeugerlängsachse 14 stehen, 30°. Die Längsausdehnung 1 senkrecht zur Ultraschallerzeugerlängsachse 14 beträgt, wie im Detail A dargestellt, 1 = 1,6 mm.

Die Aufhängung des Flansches ist im Wesentlichen "symmetrisch", d.h. nicht nur die Dichtungen sind gleichartig ausgeführt, sondern auch die korrespondierenden Schrägflächen. Außerdem ist der gesamte Aufbau rotationssymmetrisch aufgebaut. Mit der vorliegenden Anordnung wird erreicht, dass eine Aufhängung des Ultraschallerzeugers ohne unnötigen Energieverlust gewährleistet ist. Zum einen durch die Anordnung des Flansches in der Nulllage der Längsschwingung werden störende Eigenbewegungen dieses Flansches minimiert, zudem wird durch die Festklemmung des Flansches mit den Schrägflächen bezüglich der Dichtungen 7 und 8 eine Relativbewegung ermöglicht. Auf eine direkte Anbindung des Flansches zu dem Gehäuse wird (abgesehen von der optionalen Verdrehsicherung) vollkommen verzichtet.

Der oben dargestellte Sachverhalt wird nun nochmals mit etwas anderen Worten zusammengefasst.

Die Behandlungseinheit für die manuelle Anwendung besteht aus einem einteiligen, topfförmigen Kunststoffgehäuse und einem darin befindlichen Ultraschallerzeuger. Der Ultraschallerzeuger ist als DML-System ausgeführt und setzt sich zusammen aus einem schallaktiven Unterteil, der Gegenmasse und ein oder mehreren Piezokeramiken, die miteinander verschraubt sind.

Das topfförmige Kunststoffgehäuse ist vorzugsweise aus einem für medizinische Anwendungen zugelassenem Kunststoff, z. B. Delrin (POM), gefertigt und enthält in seinem oberen Teil eine Bohrung mit Zugentlastung für die Zuführung der elektrischen Versorgungsleitung für den Ultraschallerzeuger. Der Flansch ist Teil des schallaktiven Unterteiles und dient der Befestigung des Ultraschallerzeugers im topfförmigen Gehäuse. Er ist, bezogen auf das DML-System in einem für die Längsauslenkung dehnungsfreien Bereich angeordnet, so dass er selbst im schwingenden Betrieb keine störenden Auslenkungen vollführt. Der Flansch wird zum Außenrand hin dünner und weist rundherum, beidseitig abgeflachte Schrägen mit der Teillänge 1 und einem Winkel α kleiner 45° auf.

Im unteren Bereich des topfförmigen Gehäuses ist eine ringförmige Nut eingearbeitet, in die ein innerer und ein äußerer O-Ring eingelegt sind und zwischen denen der Flansch über seinen Außenrand fixiert und eingepresst ist. Eine Einfräsung im Flansch und ein im oberen Bereich der Nut eingelassener Dorn verhindern zusätzlich ein gewaltsames Verdrehen des Ultraschallerzeugers im Gehäuse.

Das schallaktive Unterteil des Ultraschallerzeugers ist aus einem biokompatiblen Material ausgeführt, vorzugsweise aus einer Titanlegierung.

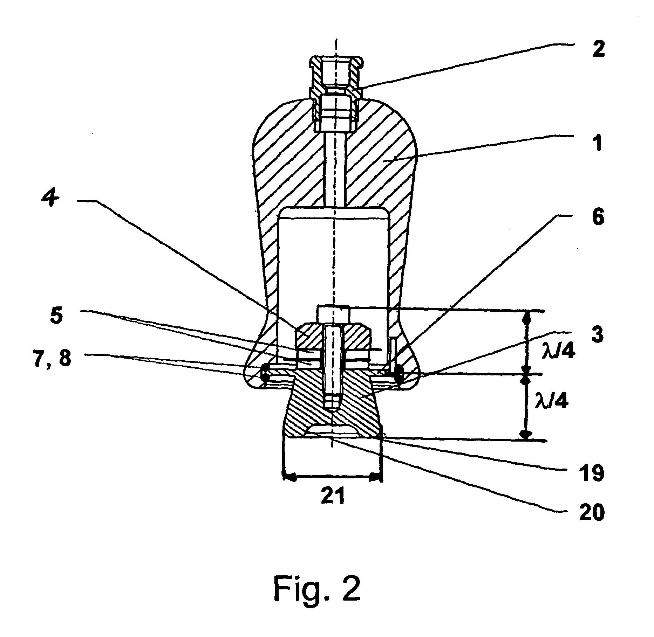

Fig. 2 zeigt eine weitere Ausführungsform einer Behandlungseinheit (Handapplikator), welche sich von der vorgenannten Ausführungsform jedoch primär durch eine andere Gestaltung des Unterteils abhebt. Soweit nicht auf Unterschiede zu der vorherigen Ausführungsform eingegangen wird, sind die Verhältnisse mit der in Fig. 1 gezeigten Ausführungsform gleich.

Der in Fig. 2 gezeigte Ultraschallerzeuger unterscheidet sich im Wesentlichen durch eine andere Gestaltung des Unterteils 3 von der in Fig. 1 gezeigten Ausführungsform.

Zunächst einmal ist die Länge des Unterteils eine andere, es handelt sich hier um ein Unterteil mit einer Länge von ca. λ/4, so dass sich für den gesamten Ultraschallerzeuger eine Länge von λ/2 ergibt. Hierdurch ist ein Schwingungsnulldurchgang im Bereich λ/4, also der Einspannung zwischen den beiden "O-Ringen" gegeben. Hierauf wurde oben bereits Bezug genommen.

Das schallaktive Unterteil 3 ist aus einem biokompatiblen Material, vorzugsweise einer Titanlegierung. Der Kontaktbereich (es handelt sich im vorliegenden Beispiel um eine im Wesentlichen kreisringförmige Ausführung) ist mit dem Bezugszeichen 21 bezeichnet.

Der Kontaktbereich weist eine muldenartige Ausarbeitung 20 auf, welche sich in Richtung der Längsachse des Ultraschallerzeugers um 1/5 der Länge des Unterteils 3 hin nach oben erstreckt.

Der Durchmesser der muldenförmigen Ausarbeitung beträgt hierbei 2/3 des Außendurchmessers des Unterteils (siehe Fig. 2). Für eine gute Handhabung und Reinigung des Unterteils weisen die stegförmige Struktur 19, welche die muldenförmige Ausarbeitung umgibt sowie die muldenförmige Ausarbeitung 20 selbst entsprechende Radien auf.

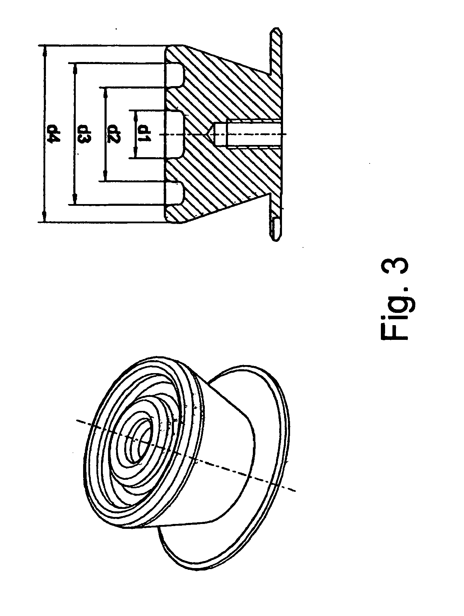

Fig. 3 zeigt eine weitere Ausführungsform eines Unterteils.

Dieses weist konzentrische stegförmige Strukturen auf, wobei die innere Struktur einen Ring mit einem Innendurchmesser D1 und einem Außendurchmesser D2 beschreibt sowie die äußere Struktur einen Ring mit einem inneren Durchmesser D3 sowie einem äußeren Durchmesser D4. Im vorliegenden Beispiel sind die Größenbeziehungen zwischen den einzelnen Durchmessern wie folgt: d2= 2xd1, d3 = 3xd1, d4 = 3,5xd1.

Diese Verhältnisse sind nicht zwingend. Es sind hiervon auch Abweichungen möglich. Insbesondere sind auch Abweichungen von den rotationssymmetrischen Ausführungen der stegförmigen Strukturen möglich, so sind insbesondere Ellipsoide, rechteckige oder auch dreieckige Gestaltungen möglich.

Der Betrieb aller in Fig. 1 bis Fig. 3 gezeigten Gegenstände erfolgt vorzugsweise mit Ultraschall im Bereich von 30 - 120 kHz, insbesondere bevorzugt im Bereich von 40 - 60 kHz. Hierbei wird vorzugsweise gepulste Hochfrequenzenergie eingebracht mit einer Pulsationsfrequenz von z.B. 30 Hz. Die Einschaltzeit der Einleitung der gepulsten Hochfrequenzenergie beträgt beispielsweise 20% der Ausschaltzeit.

Die oben genannten Ausführungsbeispiele wurde zu Erläuterungszwecken gegeben, diese sollen keinerlei Einschränkung der in der vorliegenden Patentanmeldung beschriebenen allgemeinen Lehre bzw. der Patentansprüche darstellen.

### Bezugszeichenliste

- 1: topfförmiges Gehäuse
- 2: Durchführung mit Zugentlastung für die elektrische Zuleitung
- 3: Unterteil des Ultraschallerzeugers
- 4: Oberteil des Ultraschallerzeugers (Gegenmasse)
- 5: Piezoelement
- 6: Flansch des Ultraschallerzeugers
- 7: Dichtung (innerer O-Ring)
- 8: Dichtung (äußerer O-Ring)
- 9: Aufnahme (ringförmige Nut im topfförmigen Gehäuse)
- 10: Ausfräsung im Flansch
- 11: Dorn zur Fixierung des Ultraschallerzeugers
- 12: Ultraschallerzeuger
- 13: Behandlungseinheit
- 14: Längsachse des Ultraschallerzeugers
- 15: Querschnittsebene des Ultraschallerzeugers
- 16: Schrägflächen
- 17: Schrägflächen
- 18: Nulllage
- 19, 19.1, 19.2: ringförmige Kontaktfläche des Unterteils des Ultraschallerzeugers
- 20: muldenförmige Ausarbeitung im Kontaktbereich des Ultraschallerzeugers
- 21: Kontaktbereich
- α: Winkel der Schrägen am Flansch
- 1: Länge der Schrägen am Flansch
- t: Flanschdicke
- d₁, d₂, d₃, d₄: Durchmesser stegförmiger Strukturen

## Patentansprüche

1. Behandlungseinheit (13) für therapeutische sowie in-vitro-Anwendungen, enthaltend:
ein topfförmiges Gehäuse (1) sowie einen im Wesentlichen stabförmigen Ultraschallerzeuger (12), wobei
das topfförmige Gehäuse (1) eine Aufnahme (9) mit zwei Dichtungen (7, 8) aufweist,
und der Ultraschallerzeuger (12) einen Flansch (6) aufweist, **dadurch gekennzeichnet, dass** der Flansch zumindest eine zu der Querachse (15) des Ultraschallerzeugers (12) unter einem Winkel 5° < α < 85° winklige oder qekrümmte Schrägfläche aufweist, und weiters,
dass mindestens eine Dichtung (7, 8) auf der Schrägfläche (16, 17) des Flansches (6) abgestützt ist.

2. Behandlungseinheit nach Anspruch 1, **dadurch gekennzeichnet, dass** der Flansch (6) seitlich umlaufend ist.

3. Behandlungseinheit nach einem der vorhergehenden Ansprüche, **dadurch gekennzeichnet, dass** der Flansch (6) in einer bzw. um eine Nulllage (18) der Längsschwingung des Ultraschallerzeugers (12) angeordnet ist.

4. Behandlungseinheit nach einem der vorhergehenden Ansprüche, **dadurch gekennzeichnet, dass** die Flanschdicke t abhängig von der Wellenlänge λ ist und der Ungleichung t ≤ λ/50 folgt, aber mindestens t ≥ 0,5 mm beträgt.

5. Behandlungseinheit nach einem der vorhergehenden Ansprüche, **dadurch gekennzeichnet, dass** der gemittelte Durchmesser der Flansches (6) 15% bis 300 %, bevorzugt 50 % bis 100 %, größer ist als der gemittelte Durchmesser des übrigen Ultraschallerzeugers (12) und/oder seines Unterteiles (3).

6. Behandlungseinheit nach einem der vorhergehenden Ansprüche, **dadurch gekennzeichnet, dass** die Schrägflächen (16, 17) eine Längenausdehnung senkrecht zur Längsachse des Ultraschallerzeugers von mindestens 0,5 mm aufweisen, aber nicht mehr als 45 % der Differenz des gemittelten Durchmessers des Flansches und des übrigen Ultraschallerzeugers und/oder seines Unterteiles.

7. Behandlungseinheit nach einem der vorhergehenden Ansprüche, **dadurch gekennzeichnet, dass** bei einer geradlinigen Schrägfläche (16, 17) der Winkel α zwischen Schrägfläche und der Fläche senkrecht zur Ultraschallerzeugerlängsachse α = 10° - 80°, vorzugsweise α = 30° - 60°, besonders vorzugsweise 30° - 45° beträgt.

8. Behandlungseinheit nach einem der vorhergehenden Ansprüche, **dadurch gekennzeichnet, dass** die Schrägfläche (16, 17) Stufen aufweist bzw. eine Aneinanderreihung unterschiedlicher Winkel aufweist oder gekrümmt ist bzw. sich ändernde Krümmungen aufweist.

9. Behandlungseinheit nach einem der vorhergehenden Ansprüche, **dadurch gekennzeichnet, dass** der Flansch (6) zur Minimierung der Eigenresonanz perforiert ist.

10. Behandlungseinheit nach einem der vorhergehenden Ansprüche, **dadurch gekennzeichnet, dass** eine Verdrehsicherung (10, 11) zwischen dem Ultraschallerzeuger und dem Gehäuse gegeben ist.

11. Behandlungseinheit nach einem der vorhergehenden Ansprüche, **dadurch gekennzeichnet, dass** die Aufnahme (9) nutförmig umlaufend ausgestaltet ist.

12. Behandlungseinheit nach einem der vorhergehenden Ansprüche, **dadurch gekennzeichnet, dass** die Dichtung (7, 8) vorzugsweise umlaufend ist sowie vorzugsweise einen runden, ellipsoiden oder mehreckigen Querschnitt aufweist.

13. Behandlungseinheit nach einem der vorhergehenden Ansprüche, **dadurch gekennzeichnet, dass** die Gesamtlänge des Ultraschallerzeugers (12) ein ganzzahliges Vielfaches von λ/2 ist.

14. Behandlungseinheit nach einem der vorhergehenden Ansprüche, **dadurch gekennzeichnet, dass** der Ultraschallerzeuger (12) mehrteilig ausgeführt ist, wobei ein oder mehrere, vorzugsweise zwei scheibenförmige Piezoelemente (5) zwischen einem Oberteil (4) und einem zur Berührung mit dem zu behandelnden Objekt in Kontakt zu bringendes Unterteil (3) vorgesehen sind.

15. Behandlungseinheit nach Anspruch 14, **dadurch gekennzeichnet, dass** das Unterteil (3) in seinem Kontaktbereich (21) zum zu behandelnden Objekt eine zumindest bereichsweise von einer stegförmigen Struktur (19) umgebene muldenartige Ausarbeitung (20) aufweist, welche sich in Längsachse (14) des Ultraschallerzeugers vorzugsweise im Bereich von 1/4 bis 1/6 der Länge des Unterteils (3) erstreckt.

16. Behandlungseinheit nach Anspruch 15, **dadurch gekennzeichnet, dass** die Projektionsfläche der muldenartigen Ausarbeitung senkrecht zur Längsachse (14) des Ultraschallerzeugers das 0,1-fache bis 0,5-fache des Kontaktbereichs (21) des Ultraschallerzeugers aufweist.

17. Behandlungseinheit nach einem der Ansprüche 15 oder 16, **dadurch gekennzeichnet, dass** mehrere, vorzugsweise sich umschließende, insbesondere konzentrische, und durch dazwischen liegende muldenartige Ausarbeitungen getrennte stegförmige Strukturen (19.1, 19.2) gegeben sind.

18. Behandlungseinheit nach einem der Ansprüche 15 - 17, **dadurch gekennzeichnet, dass** die stegförmigen Strukturen (19; 19.1, 19.2) im Wesentlichen ellipsoid, rechteckig oder dreieckig in der Ebene senkrecht zur Längsachse (14) des Ultraschallerzeugers ausgeführt sind.

19. Behandlungseinheit nach einem der vorhergehenden Ansprüchen, **dadurch gekennzeichnet, dass** der Ultraschallerzeuger mit gepulster Hochfrequenzenergie anregbar ist und die Pulsationsfrequenz der Hochfrequenzenergie im Bereich von 01 - 100 Hz, bevorzugt zwischen 08 - 40 Hz, liegt.

20. Behandlungseinheit nach einem der vorhergehenden Ansprüchen, **dadurch gekennzeichnet, dass** das die Einschaltzeit kleiner als die Ausschaltzeit der gepulsten Hochfrequenzenergie ist und die Einschaltzeit bevorzugt 10% - 50% der Ausschaltzeit beträgt.

21. Behandlungseinheit nach einem der vorhergehenden Ansprüchen, **dadurch gekennzeichnet, dass** die Frequenz der dem Ultraschallerzeuger zugeführten Hochfrequenzenergie im Bereich von 30 - 120 kHz, bevorzugt von 40 - 60 kHz, liegt.

## Claims

1. A treatment unit (13) for therapeutic and in vitro applications, comprising: a pot-shaped housing (1) and a substantially rod-shaped ultrasound generator (12), the pot-shaped housing (1) having a receiver (9) with two seals (7, 8), and the ultrasound generator (12) having a flange (6), **characterised in that** the flange has at least one inclined surface which is curved or angular to the transverse axis (15) of the ultrasound generator (12) at an angle of 5° <α< 85° and furthermore **in that** at least one seal (7, 8) is supported on the inclined surface (16, 17) of the flange (6).

2. A treatment unit according to claim 1, **characterised in that** the flange (6) is laterally circumferential.

3. A treatment unit according to any one of the preceding claims, **characterised in that** the flange (6) is arranged at or around a zero position (18) of the longitudinal oscillation of the ultrasound generator (12).

4. A treatment unit according to any one of the preceding claims, **characterised in that** the flange thickness t is dependent on the wave length λ and follows the inequality t ≤ λ/50, however is at least t ≥ 0.5 mm.

5. A treatment unit according to any one of the preceding claims, **characterised in that** the average diameter of the flange (6) is 15% to 300%, preferably 50% to 100%, greater than the average diameter of the remaining ultrasound generator (12) and/or its lower part (3).

6. A treatment unit according to any one of the preceding claims, **characterised in that** the inclined surfaces (16, 17) have a linear extension perpendicular to the longitudinal axis of the ultrasound generator of at least 0.5 mm, not however more than 45% of the difference between the average diameter of the flange and that of the remaining ultrasound generator and/or its lower part.

7. A treatment unit according to any one of the preceding claims, **characterised in that** in the case of a rectilinear inclined surface (16, 17), the angle α between the inclined surface and the surface perpendicular to the longitudinal axis of the ultrasound generator is α = 10° - 80°, preferably α = 30° - 60°, in particular preferably 30° - 45°.

8. A treatment unit according to any one of the preceding claims, **characterised in that** the inclined surface (16, 17) has steps or a stringing together of different angles or is curved or has altering curves.

9. A treatment unit according to any one of the preceding claims, **characterised in that** the flange (6) is perforated to minimise the self resonance.

10. A treatment unit according to any one of the preceding claims, **characterised in that** an anti-twist means (10, 11) is provided between the ultrasound generator and the housing.

11. A treatment unit according to any one of the preceding claims, **characterised in that** the receiver (9) is circumferential in the manner of a groove.

12. A treatment unit according to any one of the preceding claims, **characterised in that** the seal (7, 8) is preferably circumferential and preferably has a round, ellipsoidal or polygonal cross-section.

13. A treatment unit according to any one of the preceding claims, **characterised in that** the total length of the ultrasound generator (12) is an integral multiple of λ/2.

14. A treatment unit according to any one of the preceding claims, **characterised in that** the ultrasound generator (12) is multipart, one or a plurality, preferably two, disc-shaped piezo elements (5) being provided between an upper part (4) and a lower part (3) to be brought into contact with the object to be treated.

15. A treatment unit according to claim 14, **characterised in that** in its contact region (21) with the object to be treated, the lower part (3) has a trough-like formation (20) at least portions of which are surrounded by a fillet-like structure (19) and which in the longitudinal axis (14) of the ultrasound generator preferably extends in the range of 1/4 to 1/6 of the length of the lower part (3).

16. A treatment unit according to claim 15, **characterised in that** the projection surface of the trough-like formation perpendicular to the longitudinal axis (14) of the ultrasound generator has 0.1 times to 0.5 times the contact region (21) of the ultrasound generator.

17. A treatment unit according to one of claims 15 and 16, **characterised in that** a plurality of, preferably encircling, in particular concentric, fillet-like structures (19.1, 19.2) separated by trough-like formations lying therebetween are provided.

18. A treatment unit according to any one of claims 15 - 17, **characterised in that** the fillet-like structures (19; 19.1, 19.2) are substantially ellipsoidal, rectangular or triangular in the plane perpendicular to the longitudinal axis (14) of the ultrasound generator.

19. A treatment unit according to any one of the preceding claims, **characterised in that** the ultrasound generator can be excited with pulsed high-frequency energy and the pulsation frequency of the high-frequency energy lies in the range of 01 -100 Hz, preferably between 08 - 40 Hz.

20. A treatment unit according to any one of the preceding claims, **characterised in that** the on period is shorter than the off period of the pulsed high-frequency energy and the on period is preferably 10% - 50% of the off period.

21. A treatment unit according to any one of the preceding claims, **characterised in that** the frequency of the high-frequency energy supplied to the ultrasound generator lies in the range of 30 - 120 kHz, preferably 40 - 60 kHz.

## Revendications

1. Unité de traitement (13) pour des applications thérapeutiques ainsi que des applications *in vitro,* comprenant :
un boîtier en forme de pot (1) ainsi qu'un générateur d'ultrasons (12) substantiellement en forme de barre, dans laquelle
le boîtier en forme de pot (1) présente un logement (9) avec deux joints (7, 8), et
le générateur d'ultrasons (12) présente une bride (6),
**caractérisée en ce que** la bride présente au moins une surface inclinée courbe ou angulaire sous un angle de 5° < α < 85° par rapport à l'axe transversal (15) du générateur d'ultrasons (12), et en outre,
**en ce qu'**au moins un joint (7, 8) repose sur la surface inclinée (16, 17) de la bride (6).

2. Unité de traitement selon la revendication 1, **caractérisée en ce que** la bride (6) est latéralement périphérique.

3. Unité de traitement selon l'une quelconque des revendications précédentes, **caractérisée en ce que** la bride (6) est disposée dans une position zéro (18), ou autour de celle-ci, de l'oscillation longitudinale du générateur d'ultrasons (12).

4. Unité de traitement selon l'une quelconque des revendications précédentes, **caractérisée en ce que** l'épaisseur de bride t dépend de la longueur d'onde λ et se conforme à l'inégalité t ≤ λ/50, mais s'élève au moins à t ≥ 0,5 mm.

5. Unité de traitement selon l'une quelconque des revendications précédentes, **caractérisée en ce que** le diamètre moyen de la bride (6) est de 15 % à 300 %, de préférence de 50 % à 100 %, supérieur au diamètre moyen du reste du générateur d'ultrasons (12) et/ou de sa partie inférieure (3).

6. Unité de traitement selon l'une quelconque des revendications précédentes, **caractérisée en ce que** les surfaces inclinées (16, 17) présentent une étendue longitudinale perpendiculaire à l'axe longitudinal du générateur d'ultrasons d'au moins 0,5 mm mais de pas plus de 45 % de la différence entre le diamètre moyen de la bride et du reste du générateur d'ultrasons et/ou de sa partie inférieure.

7. Unité de traitement selon l'une quelconque des revendications précédentes, **caractérisée en ce que** pour l'une des surfaces inclinées rectilignes (16, 17), l'angle α entre la surface inclinée et la surface perpendiculaire à l'axe longitudinal du générateur d'ultrasons est α = 10° à 80°, de préférence α = 30° à 60°, de plus grande préférence α = 30° à 45°.

8. Unité de traitement selon l'une quelconque des revendications précédentes, **caractérisée en ce que** la surface inclinée (16, 17) présente des gradins ou présente une suite d'angles différents ou est courbée ou présente des courbures variables.

9. Unité de traitement selon l'une quelconque des revendications précédentes, **caractérisée en ce que** la bride (6) est perforée pour minimiser la résonance propre.

10. Unité de traitement selon l'une quelconque des revendications précédentes, **caractérisée en ce qu'**un dispositif antirotation (10, 11) existe entre le générateur d'ultrasons et le boîtier.

11. Unité de traitement selon l'une quelconque des revendications précédentes, **caractérisée en ce que** le logement (9) est conformé de façon périphérique en forme de rainure.

12. Unité de traitement selon l'une quelconque des revendications précédentes, **caractérisée en ce que** le joint (7, 8) est de préférence périphérique et présente de préférence une section transversale ronde, ellipsoïde ou polygonale.

13. Unité de traitement selon l'une quelconque des revendications précédentes, **caractérisée en ce que** la longueur totale du générateur d'ultrasons (12) est un multiple entier de λ/2.

14. Unité de traitement selon l'une quelconque des revendications précédentes, **caractérisée en ce que** le générateur d'ultrasons (12) est réalisé en plusieurs pièces, dans laquelle un ou plusieurs, de préférence deux, éléments piézoélectriques (5) en forme de disque sont prévus entre une partie supérieure (4) et une partie inférieure (3) à mettre en contact avec un objet à traiter.

15. Unité de traitement selon la revendication 14, **caractérisée en ce que** la partie inférieure (3) présente dans sa zone de contact (21) avec l'objet à traiter une particularité (20) de type cavité, entourée au moins par endroits d'une structure en forme de barrette (19), la particularité s'étendant dans l'axe longitudinal (14) du générateur d'ultrasons de préférence au niveau de 1/4 à 1/6 de la longueur de la partie inférieure (3).

16. Unité de traitement selon la revendication 15, **caractérisée en ce que** la surface de projection de la particularité de type cavité présente perpendiculairement à l'axe longitudinal (14) du générateur d'ultrasons 0,1 à 0,5 fois la zone de contact (21) du générateur d'ultrasons.

17. Unité de traitement selon l'une quelconque des revendications 15 ou 16, **caractérisée en ce qu'**il existe plusieurs structures en forme de barrettes (19.1, 19.2), de préférence s'entourant mutuellement, en particulier concentriques, et séparées par des particularités de type cavité situées entre celles-ci.

18. Unité de traitement selon l'une quelconque des revendications 15 à 17, **caractérisée en ce que** les structures en forme de barrettes (19 ; 19.1, 19.2) sont réalisées de façon substantiellement ellipsoïde, rectangulaire ou triangulaire dans le plan perpendiculaire à l'axe longitudinal (14) du générateur d'ultrasons.

19. Unité de traitement selon l'une quelconque des revendications précédentes, **caractérisée en ce que** le générateur d'ultrasons peut être excité par une énergie pulsée haute fréquence et la fréquence de pulsation de l'énergie haute fréquence se situe dans la plage de 01 à 100 Hz, de préférence entre 08 à 40 Hz.

20. Unité de traitement selon l'une quelconque des revendications précédentes, **caractérisée en ce que** le temps de mise sous tension est inférieur au temps de mise hors tension de l'énergie pulsée haute fréquence et le temps de mise sous tension correspond de préférence à 10 % à 50 % du temps de mise hors tension.

21. Unité de traitement selon l'une quelconque des revendications précédentes, **caractérisée en ce que** la fréquence de l'énergie haute fréquence amenée au générateur d'ultrasons se situe dans la plage de 30 à 120 kHz, de préférence de 40 à 60 kHz.
